# EUROPEAN PATENT APPLICATION

(11) **EP 3 939 586 A1**
(43) Date of publication of application: **19.01.2022**
(21) Application number: 21192564.9
(22) Date of filing: 27.08.2014
(51) Int. Cl.: A61K 31/423, A61K 31/202, A61K 31/232, A61P 3/06, A61P 9/10, A61P 43/00

(54) **COMBINATION OF PEMAFIBRATE AND OMEGA-3 FATTY ACIDS FOR DYSLIPEDMIA**

(30) Priority: 28.08.2013 JP 2013176364
(62) Divisional of application: 14841322.2
(71) Applicant: KOWA COMPANY, LTD., Naka-ku Nagoya-shi Aichi 460-8625 (JP)
(72) Inventor: TAKIZAWA, Toshiaki, Tokyo 189-0022 (JP)
(74) Representative: Weist, Stefan

(57) **Abstract**

This invention provides a combination-drug composition and a combination use of pharmaceuticals for preventing and/or treating dyslipidemia states, such as hyper-LDL cholesterolemia, in mammals, including humans. This invention pertains to a drug composition for preventing and/or treating dyslipidemia and the like, said drug composition comprising the following:
(R)-2-[3-[[N-(benzoxazole-2-yl)-N-3-(4-methoxyphenoxy)propy 1]aminomethyl]phenoxy] butyric acid, a salt thereof, or a solvate of either; and an omega-3 fatty acid or an ester derivative of an ω-3 fatty acid.

## Description

### Technical Field

The present invention relates to a composition containing (R)-2-[3-[[N-(benzoxazol-2-yl)-N-3-(4-methoxyphenoxy)propyl ]aminomethyl]phenoxy]butyric acid and ω-3 fatty acid(s) or ester derivative(s) thereof, which are intended to prevent and/or treat atherosclerosis and lipidemia symptoms such as hypercholesterolemia and the like, and combination use thereof.

### Background Art

In recent years, due to westernization of diet, patients with hypercholesterolemia, hypertriglyceridemia, hypo-HDL cholesterolemia and the like, which are deemed to fall within so-called lifestyle-related diseases, are in an increasing trend. Furthermore, patients with mixed or combined dyslipidemia having both hypercholesterolemia and hypertriglyceridemia are increasing recently. Specifically, LDL cholesterol (LDL-C) and triglyceride (TG) have raised and HDL cholesterol (HDL-C) has decreased in a patient with mixed dyslipidemia, and such high TG and low HDL-C condition is also observed in patients with metabolic syndrome or diabetes mellitus. It has been proved that hyper-LDL cholesterolemia, hypo-HDL cholesterolemia and hypertriglyceridemia are risk factors for coronary artery diseases (CADs), cerebrovascular disorders and the like, and the importance of the management of these kinds of dyslipidemia is also described in "Guidelines for Prevention of Atherosclerotic Cardiovascular Diseases 2012" by Japan Atherosclerosis Society.

Dyslipidemia, in particular, hypercholesterolemia has already fallen into a disease area of high medical satisfaction with the advent of statins . However, from the results of a large number of large-scale clinical trials, it has been found that the further decrease of blood LDL cholesterol level leads to the prevention of coronary artery diseases (the lower the better), and the more strict lipid control is recommended. There are a large number of patients who cannot achieve the targeted level of blood LDL-C only by the statins, and thus combination use of multiple agents has been required (Non-Patent Document 1).

PPAR is one of receptors that belong to a nuclear receptor family. As these receptors, the existence of three subtypes (α, γ, and δ) are known (Non-Patent Document 2). Among them, PPARα is mainly expressed in the liver, and when PPARα is activated, the production of apo C-III is suppressed, then lipoprotein lipase (LPL) is activated, and fats are consequently catabolized. As PPARα agonists, unsaturated fatty acids, fibrate-based agents such as fenofibrate, bezafibrate and gemfibrozil, and the like are known (Non-Patent Document 3). Furthermore, compounds having a stronger and more selective action to activate PPARα than the actions of conventional fibrate-based drugs has been reported in recent years (Patent Documents 1 to 10).

Omega-3 fatty acids typified by eicosapentaenoic acid (EPA) and docosahexaenoic acid (DHA) are long-chain essential fatty acids that are mainly contained in fish oil. It is reported that the fatty acids decrease blood triglyceride level by suppressing the absorption of triglycerides from the intestinal tracts, suppressing biosynthesis of triglycerides in the liver, and enhancing the activity of plasma lipoprotein lipase (Non-Patent Documents 4 and 5), and decrease the total blood cholesterol level by suppressing the synthesis of hepatic cholesterol and accelerating the elimination of cholesterol bile (Non-Patent Document 6).

Under such situation, there are reports aiming at improving dyslipidemia by using a PPAR agonist and ω-3 fatty acid(s) in combination. For example, it has been reported that the action of suppressing increase of a body weight and the action of decreasing a plasma insulin level in KK mice by administrating EPA-rich or DHA-rich fish oil thereto is enhanced by combining fenofibrate administration (Non-Patent Document 7). Furthermore, in a test in which a comparison was made by using C57Bl/6J mice according to the presence or absence of the effect of combination use of either of EPA-rich or DHA-rich fish oil and fenofibrate, no difference was observed in both cases in the suppression of lipogenesis or acceleration of fatty acid oxidation, whereas with respect to the acceleration of cholesterol catabolism (enhancement of the expression of CYP7A1 mRNA), a stronger interactive combination effect was exerted in the combination use of EPA-rich fish oil and fenofibrate than in the combination use of DHA-rich fish oil and fenofibrate (Non-Patent Document 8). However, in the data, the total cholesterol levels reported in these two documents were higher in the combination use groups, and the action of decreasing LDL cholesterol level cannot be confirmed. On the other hand, in a clinical example of administration to a patient with familial hypertriglyceridemia, it was reported that the total cholesterol level was reduced by 46% by administering a combination of fenofibrate and Omacor for a month (Patent Document 11). However, the disclosed data is data of dosing to a patient with acute pancreatitis as a background, and there is no comparison with the effect of single administration of fenofibrate or an ω-3 fatty acid. In addition, any action of decreasing LDL cholesterol level was not confirmed.

### Citation List

### Patent Document

Patent Document 1: WO 2005/023777 A1
Patent Document 2: WO 2009/080248 A1
Patent Document 3: WO 2009/047240 A1
Patent Document 4: WO 2008/006043 A2
Patent Document 5: WO 2006/049232 A1
Patent Document 6: WO 2006/033891 A2
Patent Document 7: WO 2005/009942 A1
Patent Document 8: WO 2004/103997 A1
Patent Document 9: WO 2005/097784 A1
Patent Document 10: WO 2003/043997 A1
Patent Document 11: JP 2008-524120 A

### Non-Patent Document

Non-Patent Document 1: Folia Pharmacol. Jpn., 129, 267-270 (2007)
Non-Patent Document 2: J. Lipid Research, 37, 907-925 (1996)
Non-Patent Document 3: Trends in Endocrinology and Metabolism, 15 (7), 324-330 (2004)
Non-Patent Document 4: Eur. J. Pharmacol., 235, 221-227 (1993)
Non-Patent Document 5: Atherosclerosis, 18 (5), 536 (1990)
Non-Patent Document 6: Eur. J. Pharmacol., 231, 121-127 (1993)
Non-Patent Document 7: J. Atheroscler. Thromb., 16 (5), 674-683 (2009)
Non-Patent Document 8: J. Atheroscler. Thromb., 16 (3), 283-291 (2009)

### Summary of the Invention

### Problems to be Solved by the Invention

The problem of the present invention is to provide a pharmaceutical composition comprising a combination of drugs and combination use of agents for preventing and/or treating atherosclerosis and dyslipidemic conditions such as hypercholesterolemia, hyper-LDL cholesterolemia and hypertriglyceridemia.

### Means to Solving the Problems

In view of such circumstances, the present inventors did intensive studies, and consequently found the fact that a potent blood LDL cholesterol decreasing effect is exerted by a combination of (R)-2-[3-[[N-(benzoxazol-2-yl)-N-3-(4-methoxyphenoxy)propyl ]aminomethyl]phenoxy]butyric acid (Example 85 of Patent Document 1), or a salt thereof, with EPA that is an ω-3 fatty acid, wherein the (R)-2-[3-[[N-(benzoxazol-2-yl)-N-3-(4-methoxyphenoxy)propyl ]aminomethyl]phenoxy]butyric acid is disclosed in the Patent Document 1 to have a selective PPARα activating effect, and to be useful as a prophylactic and/or therapeutic agent for dyslipidemia, arteriosclerosis, diabetes, diabetic complication (diabetic nephropathy and the like), inflammation, heart disease, and the like without association of weight gain or obesity in mammals including human beings, and thus have completed the present invention.

That is, the present invention provides a pharmaceutical composition for preventing and/or treating dyslipidemia, comprising:
a) (R)-2-[3-[[N-(benzoxazol-2-yl)-N-3-(4-methoxyphenoxy)propyl ]aminomethyl]phenoxy]butyric acid (hereinafter, also referred to as Compound A), or a salt, or a solvate, or a solvate of the salt thereof, and
b) ω-3 fatty acid(s) or ester derivative(s) thereof.

More specifically, a pharmaceutical composition for preventing and/or treating hyper-LDL cholesterolemia comprising:
a) (R)-2-[3-[[N-(benzoxazol-2-yl)-N-3-(4-methoxyphenoxy)propyl ]aminomethyl]phenoxy]butyric acid, or a salt, or a solvate, or a solvate of the salt thereof, and
b) ω-3 fatty acid(s) or ester derivative(s) thereof, is provided.

The detailed description of the present invention is as follows.
(1) A pharmaceutical composition for preventing and/or treating dyslipidemia, comprising:
   (R)-2-[3-[[N-(benzoxazol-2-yl)-N-3-(4-methoxyphenoxy)propyl ]aminomethyl]phenoxy]butyric acid, or a salt, or a solvate, or a solvate of the salt thereof, and ω-3 fatty acid(s) or ester derivative(s) thereof.
(2) The pharmaceutical composition according to above-mentioned (1), wherein the ω-3 fatty acid is eicosapentaenoic acid (EPA).
(3) The pharmaceutical composition according to above-mentioned (1), wherein the ester derivative of the ω-3 fatty acid is ethyl eicosapentate.
(4) The pharmaceutical composition according to any one of above-mentioned (1) to (3), wherein the dyslipidemia is hyper-LDL cholesterolemia and/or hypertriglyceridemia.
(5) The pharmaceutical composition according to any one of above-mentioned (1) to (4), wherein the mass ratio of the Compound A, or a salt, or a solvate, or a solvate of the salt thereof to the ω-3 fatty acid(s) or the ester derivative(s) thereof is from 1 : 2,000 to 1 : 30,000.
(6) A pharmaceutical composition for decreasing LDL cholesterol (LDL-C) level and/or triglyceride (TG) level, comprising Compound A, or a salt, or a solvate, or a solvate of the salt thereof, and ω-3 fatty acid (s) or ester derivative (s) thereof.
(7) The pharmaceutical composition according to above-mentioned (6), wherein the ω-3 fatty acid is eicosapentaenoic acid (EPA).
(8) The pharmaceutical composition according to above-mentioned (7), wherein the ester derivative of the ω-3 fatty acid is ethyl eicosapentate.
(9) The pharmaceutical composition according to any one of above-mentioned (6) to (8), wherein the disease that requires decreasing of LDL cholesterol (LDL-C) level is hyper-LDL cholesterolemia.
(10) The pharmaceutical composition according to any one of above-mentioned (6) to (8), wherein the disease that requires decreasing of triglyceride (TG) level is hypertriglyceridemia.
(11) The pharmaceutical composition according to any one of above-mentioned (6) to (10), wherein the mass ratio of the Compound A, or a salt, or a solvate, or a solvate of the salt thereof to the ω-3 fatty acid(s) or the ester derivative(s) thereof is from 1 : 2,000 to 1 : 30,000.

(12) A method for preventing and/or treating dyslipidemia in a subject, comprising: administering to a subject with dyslipidemia or a subj ect with risk of dyslipidemia an effective amount of a pharmaceutical composition comprising Compound A, or a salt, or a solvate, or a solvate of the salt thereof, and ω-3 fatty acid(s) or ester derivative(s) thereof.
(13) The method according to above-mentioned (12), wherein the ω-3 fatty acid is eicosapentaenoic acid (EPA).
(14) The method according to above-mentioned (12), wherein the ester derivative of the ω-3 fatty acid is ethyl eicosapentate.
(15) The method according to any one of above-mentioned (12) to (14), wherein the dyslipidemia is hyper-LDL cholesterolemia and/or hypertriglyceridemia.
(16) The method for preventing and/or treating dyslipidemia in a subject according to any one of above-mentioned (12) to (15), wherein the mass ratio of the Compound A, or a salt, or a solvate, or a solvate of the salt thereof to the ω-3 fatty acid(s) or the ester derivative(s) thereof in the pharmaceutical composition is from 1 : 2,000 to 1 : 30,000.
(17) A method for decreasing LDL cholesterol (LDL-C) level and/or triglyceride (TG) level in a patient who needs decreasing of LDL cholesterol (LDL-C) level and/or triglyceride (TG) level, comprising: administering to the patient an effective amount of a pharmaceutical composition containing Compound A, or a salt, or a solvate, or a solvate of the salt thereof, and ω-3 fatty acid(s) or ester derivative(s) thereof.
(18) The method according to above-mentioned (17), wherein the ω-3 fatty acid is eicosapentaenoic acid (EPA).
(19) The method according to above-mentioned (17), wherein the ester derivative of the ω-3 fatty acid is ethyl eicosapentate.
(20) The method according to any one of above-mentioned (17) to (19), wherein the disease that requires decreasing of LDL cholesterol (LDL-C) level is hyper-LDL cholesterolemia.
(21) The method according to any one of above-mentioned (17) to (19), wherein the disease that requires decreasing of triglyceride (TG) level is hypertriglyceridemia.
(22) The method according to any one of above-mentioned (17) to (21), wherein the mass ratio of the Compound A, or a salt, or a solvate, or a solvate of the salt thereof to the ω-3 fatty acid(s) or the ester derivative(s) thereof in the pharmaceutical composition is from 1 : 2,000 to 1 : 30,000.

(23) Compound A, or a salt, or a solvate, or a solvate of the salt thereof for use as a pharmaceutical composition for preventing and/or treating dyslipidemia by combining with ω-3 fatty acid(s) or ester derivative(s) thereof.
(24) The Compound A, or a salt, or a solvate, or a solvate of the salt thereof for use according to above-mentioned (23), wherein the ω-3 fatty acid is eicosapentaenoic acid (EPA).
(25) The Compound A, or a salt, or a solvate, or a solvate of the salt thereof for use according to above-mentioned (23), wherein the ester derivative of the ω-3 fatty acid is ethyl eicosapentate.
(26) The Compound A, or a salt, or a solvate, or a solvate of the salt thereof for use according to any one of above-mentioned (23) to (25), wherein the dyslipidemia is hyper-LDL cholesterolemia and/or hypertriglyceridemia.
(27) The Compound A, or a salt, or a solvate, or a solvate of the salt thereof for use according to any one of above-mentioned (23) to (26), wherein the mass ratio of the Compound A, or a salt, or a solvate, or a solvate of the salt thereof to the ω-3 fatty acid(s) or the ester derivative(s) thereof in the pharmaceutical composition is from 1 : 2,000 to 1 : 30,000.
(28) Compound A , or a salt, or a solvate, or a solvate of the salt thereof for use as a pharmaceutical composition for decreasing LDL cholesterol (LDL-C) level and/or triglyceride (TG) level by combining with ω-3 fatty acid(s) or ester derivative(s) thereof.
(29) The Compound A, or a salt, or a solvate, or a solvate of the salt thereof for use according to above-mentioned (28), wherein the ω-3 fatty acid is eicosapentaenoic acid (EPA).
(30) The Compound A, or a salt, or a solvate, or a solvate of the salt thereof for use according to above-mentioned (28), wherein the ester derivative of the ω-3 fatty acid is ethyl eicosapentate.
(31) The Compound A, or a salt, or a solvate, or a solvate of the salt thereof for use according to any one of above-mentioned (28) to (30), wherein the disease that requires decreasing of LDL cholesterol (LDL-C) level is hyper-LDL cholesterolemia.
(32) The Compound A, or a salt, or a solvate, or a solvate of the salt thereof for use according to any one of above-mentioned (28) to (30), wherein the disease that requires decreasing of triglyceride (TG) level is hypertriglyceridemia.
(33) The Compound A, or a salt, or a solvate, or a solvate of the salt thereof for use according to any one of above-mentioned (28) to (32), wherein the mass ratio of the Compound A, or a salt, or a solvate, or a solvate of the salt thereof to the ω-3 fatty acid(s) or the ester derivative(s) thereof in the pharmaceutical composition is from 1 : 2,000 to 1 : 30,000.

(34) Use of Compound A, or a salt, or a solvate, or a solvate of the salt thereof for the manufacture of a pharmaceutical composition for preventing and/or treating dyslipidemia by combining with ω-3 fatty acid(s) or ester derivative(s) thereof.
(35) The use according to above-mentioned (34), wherein the ω-3 fatty acid is eicosapentaenoic acid (EPA).
(36) The use according to above-mentioned (34), wherein the ester derivative of the ω-3 fatty acid is ethyl eicosapentate.
(37) The use according to any one of above-mentioned (34) to (36), wherein the dyslipidemia is hyper-LDL cholesterolemia and/or hypertriglyceridemia.
(38) The use according to any one of above-mentioned (34) to (37), wherein the mass ratio of the Compound A, or a salt, or a solvate, or a solvate of the salt thereof to the ω-3 fatty acid(s) or the ester derivative(s) thereof in the pharmaceutical composition is from 1 : 2,000 to 1 : 30,000.
(39) Use of Compound A, or a salt, or a solvate, or a solvate of the salt thereof for the manufacture of a pharmaceutical composition for decreasing LDL cholesterol (LDL-C) level and/or triglyceride (TG) level by combining with ω-3 fatty acid(s) or ester derivative(s) thereof.
(40) The use according to above-mentioned (39), wherein the ω-3 fatty acid is eicosapentaenoic acid (EPA).
(41) The use according to above-mentioned (39), wherein the ester derivative of the ω-3 fatty acid is ethyl eicosapentate.
(42) The use according to any one of above-mentioned (39) to (41), wherein the disease that requires decreasing of LDL cholesterol (LDL-C) level is hyper-LDL cholesterolemia.
(43) The use according to any one of above-mentioned (39) to (41), wherein the disease that requires decreasing of triglyceride (TG) level is hypertriglyceridemia.
(44) The use according to any one of above-mentioned (39) to (43), wherein the mass ratio of the Compound A, or a salt, or a solvate, or a solvate of the salt thereof to the ω-3 fatty acid(s) or the ester derivative(s) thereof in the pharmaceutical composition is from 1 : 2,000 to 1 : 30,000.

### Effects of the Invention

The agent and pharmaceutical composition of the present invention have an excellent action to decrease LDL cholesterol in blood, and thus are useful for preventing and/or treating dyslipidemia, specifically hyper-LDL cholesterolemia.

### Brief Description of Drawings

FIG. 1 illustrates plasma VLDL-C levels when Compound A (0.1 mg/kg) and EPA (3, 000 mg/kg) are administered each alone or in combination.
FIG. 2 illustrates triglyceride (TG) levels when Compound A and EPA are administered each alone or in combination.
FIG. 3 illustrates plasma LDL-C levels when Compound A and EPA are administered each alone or in combination.

### Modes for Carrying Out the Invention

(R)-2-[3-[[N-(benzoxazol-2-yl)-N-3-(4-methoxyphenoxy) propyl]aminomethyl]phenoxy]butyric acid (Compound A) used in the present invention can be produced, for example, according to the method described in WO 2005/023777 A1 or the like. Further, Compound A can also be formulated according to a method described elsewhere.

Further, in the present invention, a salt of Compound A or a solvate thereof can also be used. The salt and solvate can be produced by conventional methods.

The salt of Compound A is not particularly limited as long as it is a pharmaceutically acceptable salt, and examples of the salt include, for example, an alkali metal salt such as a sodium salt and a potassium salt; an alkaline earth metal salt such as a calcium salt and a magnesium salt; an organic base salt such as an ammonium salt and a trialkylamine salt; a mineral acid salt such as a hydrochloride and a sulfate; and an organic acid salt such as an acetate, and the like.

Examples of the solvate of Compound A or solvate of the salt of Compound A include a hydrate, and an alcohol solvate (for example, an ethanol solvate), and the like.

Examples of the ω-3 fatty acids in the present invention include eicosapentaenoic acid (EPA) and docosahexaenoic acid (DHA) .

Eicosapentaenoic acid refers to All-cis-5,8,11,14,17-eicosapentaenoic acid, and can be easily obtained by hydrolyzing natural type glycerin esters obtained from a fish oil or the like and then removing the glycerin parts. Alternatively, a commercially available product can be used. Examples of commercially available products containing EPA include Epadel, which is an EPA formulation, Omacor (an EPA/DHA/Vitamin E formulation) and the like. Furthermore, the eicosapentaenoic acid may form a salt with sodium, potassium or the like.

Examples of the ester derivative of an ω-3 fatty acid include glycerin esters and C₁₋₄ alkyl esters. Examples of the C₁₋₄ alkyl ester include a methyl ester, an ethyl ester, a propyl ester, an isopropyl ester, an n-butyl ester, an isobutyl ester, a t-butyl ester and the like. A methyl ester, an ethyl ester and a propyl ester are preferably exemplified. An ethyl ester is specifically preferable.

As mentioned above, the glycerin ester can be easily extracted as a natural type glycerin ester from natural resources. On the other hand, the lower alkyl ester can be easily produced by dehydration condensation of the ω-3 fatty acid with an aliphatic lower alcohol. In addition, the purity of the above-mentioned ω-3 fatty acid (s) or ester derivative (s) thereof is not specifically limited, and those having high purity are preferable since it is sufficient to administer in a small amount.

As shown in the Examples mentioned below, by combining Compound A, or a salt, or a solvate, or a solvate of the salt thereof with the ω-3 fatty acid(s) or ester derivative(s) thereof, the lipid parameters in the plasma were improved and a specifically potent action of decreasing LDL-C level was shown in an evaluation system using Wister rats. Therefore, the agent of the present invention is useful for preventing and/or treating dyslipidemia such as hypercholesterolemia and hyper-LDL cholesterolemia.

The dyslipidemia in the present invention refers to a case when one or two or more of total triglyceride (TG) level, total cholesterol (TC) level, VLDL cholesterol (VLDL-C) level, LDL cholesterol (LDL-C) level or an HDL cholesterol (HDL-C) level in blood deviate(s) from the range(s) of normal levels. Dyslipidemia as the preferable target of the present invention includes the case when VLDL cholesterol (VLDL-C) level, LDL cholesterol (LDL-C) level or triglyceride (TG) level deviates from the range of normal levels. Furthermore, the disease for which decrease of LDL cholesterol (LDL-C) level and/or triglyceride (TG) level is required in the present invention refers to the case when LDL-C level and/or TG level in blood has/have increased to be higher than normal level(s).

The Compound A, or a salt, or a solvate, or a solvate of the salt thereof of the present invention can be prepared alone or in combination with other pharmaceutically acceptable carriers into dosage forms such as a tablet, a capsule, a granule, a powder, a lotion, an ointment, an injection or a suppository or the like. These formulations can be produced by known methods. For example, a preparation for oral administration can be produced by the formulation of a solubilizer such as tragacanth gum, gum arabic, sucrose fatty acid ester, lecithin, olive oil, soybean oil, and PEG 400; an excipient such as starch, mannitol, and lactose; a binder such as methylcellulose, carboxymethylcellulose sodium, and hydroxypropylcellulose; a disintegrant such as crystalline cellulose, and carboxymethylcellulose calcium; a lubricant such as talc, and magnesium stearate; a fluidity-improving agent such as light silicic anhydride; and the like, in appropriate combination.

As to the usage form of the pharmaceutical composition of the present invention, a) Compound A, or a salt, or a solvate, or a solvate of the salt thereof can be used in combination with b) ω-3 fatty acid(s) or ester derivative(s) thereof, and the pharmaceutical composition can be used in a form in which a prophylactic and/or therapeutic effect for dyslipidemia such as hypercholesterolemia and hyper-LDL cholesterolemia is exerted with the use of the synergistic blood HDL-C increasing effect by the administration of both agents in addition to each effect of agents, however, is not limited to these usage forms. Compound A, or a salt, or a solvate, or a solvate of the salt thereof and the ω-3 fatty acid (s) or ester derivative (s) thereof may be simultaneously administered, or may be separately administered at interval(s).

Compound A, or a salt, or a solvate, or a solvate of the salt thereof and the ω-3 fatty acid(s) or ester derivative (s) thereof may be prepared together into a single formulation, or may be prepared separately into each formulation and used as a kit. That is, the pharmaceutical composition of the present invention may be a kit composed of an agent comprising as an active ingredient at least one kind selected from Compound A, or a salt, or a solvate, or a solvate of the salt thereof, and an agent comprising at least one kind of the ω-3 fatty acid(s) or ester derivative(s) thereof, in combination.

In the present invention, in the case when the two agents are administered as a single formulation, the mixing ratio of Compound A, or a salt, or a solvate, or a solvate of the salt thereof to the ω-3 fatty acid (s) or ester derivative (s) thereof can be suitably selected within the scopes of the effective doses of the respective active ingredients, and is generally preferably in the range of from 1 : 10 to 1 : 100,000, more preferably in the range of from 1 : 2, 000 to 1 : 30, 000 on the basis of mass ratio. Specifically, in the case when the ω-3 fatty acid or ester derivative thereof is EPA, it is preferable that the ratio is 1 : 2, 000 to 1 : 30, 000 on the basis of mass ratio, since an especially excellent synergistic effect can be obtained.

In the case when Compound A, or a salt, or a solvate, or a solvate of the salt thereof and the ω-3 fatty acid(s) or ester derivative (s) thereof are separately prepared, the dosage forms of the two agents may be identical or different from each other. Furthermore, the numbers of administration of the respective components may be vary.

Compound A, or a salt, or a solvate, or a solvate of the salt thereof in the present invention may be orally or parenterally administered. The dose of the pharmaceutical of the present invention varies depending on the body weight, age, sex, symptoms and the like of a subject, and usually in a case of a general adult human, it is preferable to administer as (R)-2-[3-[[N-(benzoxazol-2-yl)-N-3-(4-methoxyphenoxy)propyl ]aminomethyl]phenoxy]butyric acid by 0.001 to 100 mg, preferably 0.1 to 0.4 mg a day while being divided into one to three times per day. Furthermore, it is preferable to administer EPA as the ω-3 fatty acid(s) or ester derivative (s) thereof by 100 to 10,000 mg, preferably 1,800 to 2, 700 mg a day while being divided into one to three times per day.

### Examples

The present invention will be described more specifically by Examples, however, the present invention should not be limited at all by these Examples.

### Example 1 Effects of combination use of Compound A and EPA on VLDL-C levels and TG levels in rats

### 1. Method

Wister rats (7-week old, male, Charles River Laboratories Japan Inc.) were used for the experiments. Under ad libitum feeding, the blood samples were obtained from the cervical vein, and the rats were divided into four groups (N = 8) based on the plasma TG levels, plasma TC levels and the body weights. From the next day, a solvent (a 0.5% aqueous solution of methyl cellulose: MC),
(R)-2-[3-[[N-(benzoxazol-2-yl)-N-3-(4-methoxyphenoxy)propyl ]aminomethyl]phenoxy]butyric acid (Compound A) and/or EPA were orally administered once a day. In the afternoon of the final administration day, the blood samples were obtained under a condition of 4 hours fasting under anesthesia with pentobarbital, and the plasma VLDL-C levels were measured according to the method by Usui et al. (Usui S et al., Clin. Chem. 46, 63-72, 2000.) by high-speed liquid chromatography. Further, the TG levels were measured by an automatic analyzer, by using a measuring reagent, Qualigent TG (Sekisui Medical Co., Ltd.). The synergistic effect was determined by using Bürgi's formula (when a relative level in a group of combined use is smaller than a value of a product of a relative level of single agent A and a relative level of single agent B, then there is a synergistic effect).

### 2. Grouping

Group 1: Control
Group 2: 0.1 mg/kg of Compound A
Group 3: 200 mg/kg of EPA
Group 4: 0.1 mg/kg of Compound A and 200 mg/kg of EPA

### 3. Statistical analysis and data processing method

The results are shown as the mean value ± standard deviation. Comparison between the control group and the drug-administration group was performed using a Dunnett's multiple comparison test, and a risk rate of less than 5% was determined to have a significant difference.

### 4. Results

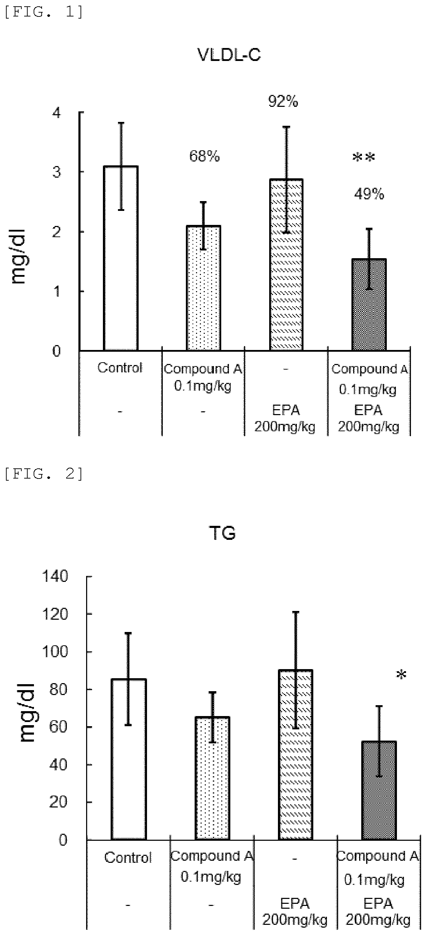

The results of the measurement of VLDL-C levels are shown in FIG. 1. Further, the relative levels of each group of VLDL-C are shown in Table 1. In the single administration of Compound A, a tendency of decreasing the level of VLDL-C was observed, and in the single administration of EPA, any obvious effect on VLDL-C level was not observed, whereas in the group of combination administration of 0.1 mg/kg of Compound A and 200 mg/kg of EPA, decrease of VLDL-C level was observed, and the decrease was significant (** : p < 0.01, to the control) and synergistic (the relative level of (0.49) in the group of combined use was smaller than the value of the product (0.68 × 0.92 = 0.63) of the relative level in the single agent A administration group to the control group and the relative level in the single agent B administration group to the control group) (FIG. 1, Table 1).

The result of the measurement of TG levels are shown in FIG. 2. Further, the relative levels of the respective groups of TG are shown in Table 2. In the single administration of Compound A, a tendency of decreasing the level of TG was observed, and in the single administration of EPA, any obvious effect on TG level was not observed, whereas in the group of combination administration of 0.1 mg/kg of Compound A and 200 mg/kg of EPA, decrease of TG level was observed, and the decrease was significant (*: p < 0.05, to the control) and synergistic (the relative level of (0.676) in the group of combined use was smaller than the value of the product (0.82 × 1.10 = 0.90) of the relative level in the single agent A administration group to the control group and the relative level in the single agent B administration group to the control group (FIG. 2, Table 2) . These results suggest that the agent and pharmaceutical composition of the present invention exert an action to improve dyslipidemia even in a patient who shows resistance against a drug therapy by a single agent.

### Synergistic VLDL-C decreasing action by combination of Compound A and EPA in rat

**[Table 1]**

| | | | | |
|---|---|---|---|---|
| Compound A (mg/kg) | | 0.1 | - | 0.1 |
| EPA (mg/kg) | | - | 200 | 200 |
| VLDL-C | Relative value (vs Control) | 0.68 | 0.92 | **0.49** |
| | Bürgi's formula | | | 0.63 |

Synergistic TG decreasing action by combination of Compound A and EPA in rat

**[Table 2]**

| | | | | |
|---|---|---|---|---|
| Compound A (mg/kg) | | 0.1 | - | 0.1 |
| EPA (mg/kg) | | - | 200 | 200 |
| TG | Relative value (vs Control) | 0.82 | 1.10 | **0.68** |
| | Bürgi's formula | | | 0.90 |

### Example 2 Effects of combination use of Compound A and EPA on LDL-C level in Wister rats

### 1. Method

Wister rats (7-week old, male, Charles River Laboratories Japan Inc.) were used for the experiments. Under ad libitum feeding, the blood samples were obtained from the cervical vein, and the rats were divided into six groups (N = 8) based on the plasma TG levels, plasma TC levels and the body weights. From the next day, a solvent (a 0.5% aqueous solution of methyl cellulose: MC),
(R)-2-[3-[[N-(benzoxazol-2-yl)-N-3-(4-methoxyphenoxy)propyl ]aminomethyl]phenoxy]butyric acid (Compound A) and/or EPA were orally administered once a day. In the afternoon of the final administration day, the blood samples were obtained under a condition of 4 hours fasting under anesthesia with pentobarbital, and the plasma LDL-C levels were measured according to the method by Usui et al. (Usui S et al., Clin. Chem. 46, 63-72, 2000.) by high-speed liquid chromatography. The synergistic effect was determined by using Bürgi' s formula (when a relative level in a group of combined use is smaller than a value of a product of a relative level of single agent A and a relative level of single agent B, then there is a synergistic effect).

### 2. Grouping

Group 1: Control
Group 2: 0.1 mg/kg of Compound A
Group 3: 200 mg/kg of EPA
Group 4: 0.1 mg/kg of Compound A and 200 mg/kg of EPA
Group 5: 3,000 mg/kg of EPA
Group 6: 0.1 mg/kg of Compound A and 3,000 mg/kg of EPA

### 3. Statistical analysis and data processing method

The results are shown as the mean value ± standard deviation. Comparison between the control group and drug-administration group was performed by using a Dunnett's multiple comparison test, and a risk rate of less than 5% was determined to have a significant difference.

### 4. Results

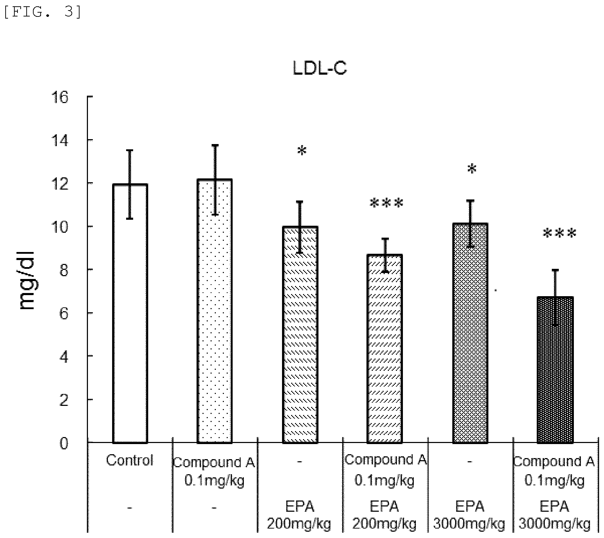

The results of the measurements of LDL-C levels after the respective drugs were administered are shown in FIG. 3. Further, the relative levels of the LDL-C in the respective groups are shown in Table 3. In the single administration of Compound A, an obvious effect on LDL-C level was not observed, whereas in the single administration of EPA, a significant (* :p < 0.05, to the control), mild decreasing action on LDL-C level was observed. However, surprisingly, in the group of combination administration of 0.1 mg/kg of Compound A and 200 mg/kg of EPA, and in the group of combination administration of 0.1 mg/kg of Compound A and 3, 000 mg/kg of EPA, decrease of LDL-C level was observed, and the decrease was significant (*** : p < 0.001, to the control) and synergistic (the relative level of (0.73) in the combination use group was smaller than the value of the product (1.02 × 0.84 = 0.86) of the relative level in the single agent A 0.1 mg/kg administration group to the control group and the relative level in the single agent EPA 200 mg/kg administration group to the control group, and the relative level of (0.56) in the combination use group was smaller than the value of the product (1.02 × 0.85 = 0.87) of the relative level in the single agent A 0.1 mg/kg administration group to the control group and the relative level in the single agent EPA 3,000 mg/kg administration group to the control group). These results suggest that the agent and pharmaceutical composition of the present invention exert an action to improve dyslipidemia even in a patient who shows a mild therapeutic effect by a drug therapy by a single agent.

Synergistic LDL-C decreasing action by combination of Compound A and EPA in high-cholesterol diet fed rats

**[Table 3]**

| | | | | | | |
|---|---|---|---|---|---|---|
| Compound A (mg/kg) | | 0.1 | - | 0.1 | - | 0.1 |
| EPA (mg/kg) | | - | 200 | 200 | 3000 | 3000 |
| LDL-C | Relative value vs. Control) ( | 1.02 | 0.84 | **0.73** | 0.85 | **0.56** |
| | Burgi's formula | | | 0.86 | | 0.87 |

### Industrial Applicability

The agent and pharmaceutical composition of the present invention have industrial applicability since they have an excellent action to decrease blood VLDL-C level and an excellent action to decrease blood LDL-C level, and thus are useful for preventing and/or treating dyslipidemia, specifically hyper-LDL cholesterolemia, hypo-HDL-cholesterolemia and hypertriglyceridemia.

## Claims

1. A pharmaceutical composition for preventing and/or treating dyslipidemia, comprising:
(R)-2-[3-[[N-(benzoxazol-2-yl)-N-3-(4-methoxyphenoxy)propyl ]aminomethyl]phenoxy]butyric acid, or a salt, or a solvate, or a solvate of the salt thereof, and an ω-3 fatty acid(s) or ester derivative(s) thereof.

2. The pharmaceutical composition according to claim 1, wherein the ω-3 fatty acid is eicosapentaenoic acid.

3. The pharmaceutical composition according to claim 1, wherein the ester derivative of the ω-3 fatty acid is ethyl eicosapentate.

4. The pharmaceutical composition according to any one of claims 1 to 3, wherein the dyslipidemia is hyper-LDL cholesterolemia and/or hypertriglyceridemia.

5. The pharmaceutical composition according to any one of claims 1 to 4, wherein the mass ratio of the (R)-2-[3-[[N-(benzoxazol-2-yl)-N-3-(4-methoxyphenoxy)propyl ]aminomethyl]phenoxy]butyric acid, or a salt, or a solvate, or a solvate of the salt thereof to the ω-3 fatty acid(s) or the ester derivative(s) thereof is from 1 : 2,000 to 1 : 30,000.

6. A pharmaceutical composition for decreasing LDL cholesterol level and/or triglyceride level, comprising: (R)-2-[3-[[N-(benzoxazol-2-yl)-N-3-(4-methoxyphenoxy)propyl ]aminomethyl]phenoxy]butyric acid, or a salt, or a solvate, or a solvate of the salt thereof, and an ω-3 fatty acid(s) or ester derivative(s) thereof.

7. The pharmaceutical composition according to claim 6, wherein the ω-3 fatty acid is eicosapentaenoic acid.

8. The pharmaceutical composition according to claim 6, wherein the ester derivative of the ω-3 fatty acid is ethyl eicosapentate.

9. The pharmaceutical composition according to any one of claims 6 to 8, wherein the disease that requires decreasing of LDL-C level is hyper-LDL cholesterolemia.

10. The pharmaceutical composition according to any one of claims 6 to 8, wherein the disease that requires decreasing of triglyceride level is hypertriglyceridemia.

11. The pharmaceutical composition according to any of claims 6 to 10, wherein the mass ratio of the
(R)-2-[3-[[N-(benzoxazol-2-yl)-N-3-(4-methoxyphenoxy)propyl ]aminomethyl]phenoxy]butyric acid, or a salt, or a solvate, or a solvate of the salt thereof to the ω-3 fatty acid(s) or the ester derivative(s) thereof is from 1 : 2,000 to 1 : 30,000.
